# NEUE EUROPÄISCHE PATENTSCHRIFT

(11) **EP 0 841 891 B2**
(45) Veröffentlichungstag und Bekanntmachung des Hinweises auf die Entscheidung über den Einspruch: **25.06.2008**
(45) Hinweis auf die Patenterteilung: 07.11.2001
(21) Anmeldenummer: 96922889.9
(22) Anmeldetag: 24.06.1996
(51) Int. Cl.: A61K 8/73, A61K 8/81, A61Q 5/06

(54) **KATIONISCHE BIOPOLYMERE ENTHALTENDE HAARKOSMETISCHE ZUBEREITUNGEN**
HAIR COSMETIC PREPARATIONS CONTAINING CATIONIC BIOPOLYMERS
PREPARATIONS CAPILLAIRES COSMETIQUES CONTENANT DES BIOPOLYMERES CATIONIQUES

(30) Priorität: 03.07.1995 DE 19524125
(43) Veröffentlichungstag der Anmeldung: 20.05.1998
(73) Patentinhaber: Cognis IP Management GmbH, 40589 Düsseldorf (DE)
(72) Erfinder: WACHTER, Rolf, D-40595 Düsseldorf (DE); KAHRE, Jörg, D-40789 Monheim (DE); HOFMANN, Matthias, D-40789 Monheim (DE); HEYER, Michael, D-40699 Erkrath (DE)
(86) Internationale Anmeldenummer: PCT/EP1996/002733
(87) Internationale Veröffentlichungsnummer: WO 1997/002007

(56) Entgegenhaltungen:
- EP-A- 0 272 472
- WO-A-95/24882
- DE-A- 2 627 419
- DE-A- 3 140 134
- DE-A- 3 302 456
- DE-A- 3 504 095
- DE-A- 3 527 974
- DE-A- 3 632 030
- DE-A- 4 234 743
- GB-A- 2 063 671
- JP-A- 1 249 712
- US-A- 4 822 598
- US-A- 5 420 197
- PARFUMERIE UND KOSMETIK, Bd. 64, Nr. 7, 1983, Seiten 367-371, XP002016101 P. GROSS ET AL: "Investigations on chitosan as a natural film forming ingredient in hair cosmetic products under the consideration of ecological aspects"
- DATABASE WPI Week 9419 Derwent Publications Ltd., London, GB; AN 94-156533 XP002016102 "Aerosol compsn having good foaming and spraying characteristics - contains spraying agent and hydroxypropyl (hydroxybutyl) chitosan , for hairdressing agent etc" & JP,A,06 100 419 (KAO) , 12.April 1994
- "Chitin und Chitosan", Proceedings of the Second International conference on Chitin and Chitosan July 12-14, 1982,S.205-209

## Beschreibung

### Gebiet der Erfindung

Die Erfindung betrifft haarkosmetische Zubereitungen mit verbessertem filmbildenden Charakter, enthaltend kationische Biopolymere ausgewählt aus der Gruppe die gebildet wird von Chitosanen, quaternierten Chitosanen, alkylierten Chitosanen und hydroxyalkylierten Chitosanen und Polyvinylpyrrolidon/Vinylacetat-Copolymere in ausgewählten Mischungsverhältnissen sowie die Verwendung der kationischen Biopolymere zur Verbesserung der filmbildenden Eigenschaften der genannten Copolymere.

### Stand der Technik

Haarkosmetische Zubereitungen, wie beispielsweise Haarlacke oder Stylingmittel, enthalten als Festigungsmittel Polymere, vorzugsweise vom Typ der Polyvinylpyrrolidon/Vinylacetate, die auf die Keratinfasern aufziehen und ihnen den gewünschten Halt verschaffen. Nachteilig ist jedoch, daß die Filme gerade bei mehrfacher Applizierung nach dem Trocknen leicht brüchig werden und dann nicht nur der Stylingeffekt verlorengeht, sondern es auch zu einer Schädigung des Haares kommen kann.

Die Aufgabe der Erfindung hat somit darin bestanden, kosmetische Mittel zur Verfügung zu stellen, die zwar als Filmbildner Polyvinylpyrrolidon/Vinylacetat-Copolymere enthalten, deren Neigung zur Spannungsrißbildung aber bei zumindest gleichbleibender Härte der Filme signifikant vermindert ist.

### Beschreibung der Erfindung

Gegenstand der Erfindung sind haarkosmetische Zubereitungen, enthaltend
a) kationische Biopolymere ausgewählt aus der Gruppe die gebildet wird von Chitosanen, quaternierten Chitosanen, alkylierten Chitosanen und hydroxyalkylierten Chitosanen und
b) Polyvinylpyrrolidon/Vinylacetat-Copolymere
im Gewichtsverhältnis 1 : 10 bis 1 : 45.

Überraschenderweise wurde gefunden, daß der Zusatz bereits sehr geringer Mengen kationischer Biopolymere ausgewählt aus der Gruppe die gebildet wird von Chitosanen, quaternierten Chitosanen, alkylierten Chitosanen und hydroxyalkylierten Chitosanen zu Polyvinylpyrrolidon/Vinylacetat-Copolymeren, deren filmbildende Eigenschaften einzeln jeweils bekannt sind, feuchtigkeitsregulierend wirkt und die Neigung zur Spannungsrißbildung in synergistischer Weise vermindert, während die Härte des Filmes nicht nachteilig beeinflußt wird. Im Hinblick darauf, daß die kationischen Biopolymere in ihrer Herstellung sehr aufwendig und daher teuer sind, besteht in der Möglichkeit, die anwendungstechnischen Eigenschaften der Copolymere durch Zusatz schon sehr geringer Mengen deutlich zu verbessern, auch eine besonderer ökonomischer Vorteil.

### Kationische Biopolymere

Geeignete kationische Biopolymere, die im Sinne der Erfindung als Komponente a) eingesetzt werden, stellen Hydrokolloide von Typ Chitosane dar. Chemisch betrachtet handelt es sich dabei um partiell deacetylierte Chitine unterschiedlichen Molekulargewichtes, die den - idealisierten - Monomerbaustein (I) enthalten:

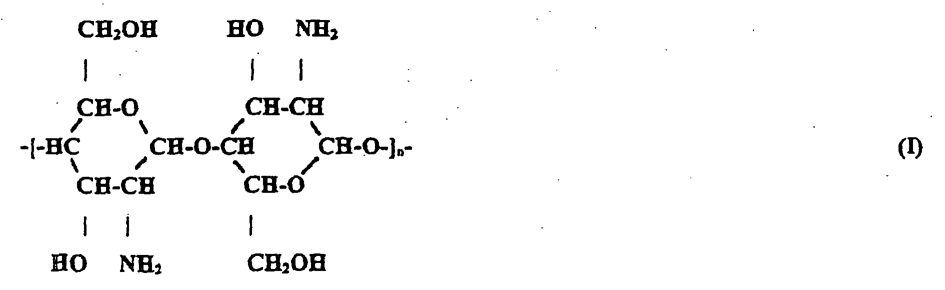

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln z.B. auch als Filmbildner eingesetzt. Neben den Chitosanen kommen auch quaternierte, alkylierte und/oder hydroxyalkylierte Derivate, gegebenenfalls auch in mikrokristalliner Form in Betracht. Der Einsatz erfolgt in der Regel in Form wäßriger Gele mit einem Feststoffgehalt im Bereich von 1 bis 5 Gew.-%.

Übersichten zu diesem Thema sind beispielsweise von B.Gesslein et al. in **HAPPI 27, 57 (1990),** O.Skaugrud in **Drug Cosm. Ind. 148, 24 (1991)** und E.Onsoyen et al. in **Seifen-Öle-Fette- Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetylien, wobei die Molekulargewichte über ein breites Spektrum verteilt sein können. Entsprechende Verfahren zur Herstellung von - mikrokristallinem - Chitosan sind beispielsweise in der WO 91/05808 (Firextra Oy) und der EP-B1 0 382 150 (Hoechst) beschrieben.

In einer bevorzugten Ausführungsform der Erfindung werden besonders aschearme kationische Biopolymere ausgewählt aus der Gruppe die gebildet wird von Chitosanen, quaternierten Chitosanen, alkylierten Chitosanen und hydroxyalkylierten Chitosanen eingesetzt, die man erhält, indem man
(a) frische Krustentierschalen mit verdünnter wäßriger Mineralsaure behandelt,
(b) das resultierende demineralisierte erste Zwischenprodukt mit waßriger Alkalihydroxidlösung behandelt,
(c) das resultierende geringfügig deproteinierte zweite Zwischenprodukt erneut mit verdünnter wäßriger Mineralsäure behandelt,
(d) das resultierende decalcifizierte dritte Zwischenprodukt schließlich mit konzentrierter wäßriger Alkalilauge behandelt und dabei bis zu einem Gehalt von 0,05 bis 0,5 und insbesondere 0,15 bis 0.25 Mol Acetamid pro Mol Monomereinheit deacetyliert, und
(e) gegebenenfalls einer Druck/Temperaturnachbehandlung zur Einstellung der Viskosität unterwirft.

### Polyvinylpyrrolidon/Vinylacetat-Copolymere

Polyvinylpyrrolidon/Vinylacetat-Copolymere (PVP/VA) der Komponente b) stellen bekannte Schutzkolloide für kosmetische Anwendungen dar, die beispielsweise unter der Marke Luviskol® von der BASF AG Ludwigshafen/FRG vertrieben werden. Üblicherweise werden die Copolymere als wäßrige Lösungen mit einem Feststoffgehalt von 1,5 bis 30 Gew.-% eingesetzt. Die Zubereitungen können die Komponenten a) und b) im Gewichtsverhältnis - jeweils bezogen auf Trockensubstanz - 1 : 10 bis 1 : 45 und insbesondere 1 : 30 bis 1 : 40 enthalten.

### Gewerbliche Anwendbarkeit

Abmischung von kationischen Biopolymeren und Polyvinylpyrrolidon/Vinylacetat-Copolymeren zeigen verbesserte filmbildende Eigenschaften. Ein weiterer Gegenstand der Erfindung betrifft daher die Verwendung von kationischen Biopolymeren als Zusatzstoffe zur Verbesserung der filmbildenden Eigenschaften von Polyvinylpyrrolidon-Vinylacetat-Copolymeren.

### Haarkosmetische Zubereitungen

Die erfindungsgemäßen Zubereitungen können als weitere Hilfs- und Zusatzstoffe Tenside, Emulgatoren, Überfettungsmittel, Verdickungsmittel, Kationpolymere, Siliconverbindungen, biogene Wirkstoffe, Filmbildner, Konservierungsmittel, Farb- und Duftstoffe enthalten.

Typische Beispiele für geeignete **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Ethercarbonsäuren, Alkyloligoglucoside, Fettsäureglucamide, Alkylamidobetaine und/oder pfanzliche Eiweißfettsäurekondensate.

Als **Emulgatoren** kommen sowohl bekannte W/O- als auch O/W- Emulgatoren wie beispielsweise gehärtetes und ethoxyliertes Ricinusöl, Polyglycerinfettsäureester oder Polyglycerinpolyricinoleate bzw. Polyglycerinpoly-12-hydroxystearate in Frage.

Als **Überfettungsmittel** können Substanzen wie beispielsweise polyethoxylierte Lanolinderivate, Lecithinderivate, Polyolfettsäureester, Monoglyceride und Fettsäurealkanol-amide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Coming, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der FR-A 22 52 840 sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A15, Mirapol® AD-1, Mirapol® AZ-1 der Miranol/US.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "Kosmetische Färbemittel" der Farbstoffkom mission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

**Filmhärte.** Zur Bestimmung der Filmhärte wurde der Meßaufbau zur Erfassung der Pendelhärte nach König verwendet (Sekunden-Pendelhärte bei 6° Auslenkung. Gerät Erichsen 299/300, Hermer/Sundvig), die als Ergebnis die Härte eines Lackfilmes wiedergibt. Dazu wurde auf einem Objektträger jeweils eine 1 Gew.-%ige Lösung eines Chitosans (Hydagen® CMF, Henkel KGaA, Düsseldorf/FRG), eines Polyvinylpyrrolidon/Vinylacetat-Copolymeres (Luviskol® VA 64, BASF AG) sowie einer Abmischung im Gewichtsverhältnis a) : b) = 1 : 40 aufgetragen und luftgetrocknet. Die Vorgehensweise wurde mehrfach wiederholt, bis ein getrockneter Film von 30 µm Dicke vorlag. Die Messung erfolgte durch Aufsetzen eines Pendels auf den Film, das mit einer konstanten Anfangsauslenkung in Schwingung versetzt wurde. Zur Meßergebnisbestimmung wurde die Anzahl der Pendelausschläge bis zur Minimalauslenkung gezählt und mit der Schwingungsdauer (1,4) multipliziert. Die Ergebnisse sind in Tabelle 1 zusammengefaßt:

**Tabelle 1**

| **Pendelhärte-Bestimmung** | | |
|---|---|---|
| **Bsp.** | **Einsatzstoff** | **Pendelhärte** |
| V1 | Chitosan | 149 |
| V2 | PVP/VA | 148 |
| 1 | Chitosan : PVP/VA = 1: 40 | 148 |

Man erkennt, daß durch Zusatz von Chitosan zum PVP/VA-Copolymer die Härte des Filmes nicht nachteilig beeinflußt wird.

**Spannungsrißbildung.** Zur Überprüfung der Spannungsrißbildung wurden Filme einer Schichtdicke von ca. 30 um nach dem Trocknen mikroskopisch untersucht. Zum Einsatz kamen PVP/VA-Copolymere mit einem Feststoffgehalt von 1,5 bis 30 Gew.-% sowie verschiedene Abmischungen von Chitosangel (1 Gew.-% Feststoffgehalt) und einer PVP/VA-Lösung (Feststoffgehalt 2,0 Gew.-%) Die Ergebnisse sind in Tabelle 2 zusammengefaßt:

**Tabelle 2**

| **Spannungsrißbildung** | | |
|---|---|---|
| **Bsp.** | **Einsatzstoff** | **Aussehen der getrockneten Filme** |
| V4 | PVP/VA Lösung (1,5 %ig) | durchgehend feine Spannungsrisse |
| V5 | PVP/VA Lösung (2,0 %ig) | durchgehend feine Spannungsrisse |
| V6 | PVP/VA Lösung (30,0 %ig) | durchgehend starke Spannungsrisse |
| 2 | Chitosan : PVP/VA = 1 : 10 | praktisch keine Spannungsrisse |
| 3 | Chitosan : PVP/VA = 1 : 20 | praktisch keine Spannungsrisse |
| 4 | Chitosan : PVP/VA = 1 : 40 | praktisch keine Spannungsrisse |
| 5 nicht gemäß der Erfindung | Chitosan : PVP/VA = 1 : 50 | praktisch keine Spannungsrisse |

## Patentansprüche

1. Haarkosmetische Zubereitungen, enthaltend
a) kationische Biopolymere ausgewählt aus der Gruppe die gebildet wird von Chitosanen, quaternierten Chitosanen, alkylierten Chitosanen und hydroxyalkylierten Chitosanen und
b) Polyvinylpyrrolidon/Vinylacetat-Copolymere
im Gewichtsverhältnis 1 : 10 bis 1 : 45.

2. Verwendung von kationischen Biopolymeren ausgewählt aus der Gruppe die gebildet wird von Chitosanen, quaternierten Chitosanen, alkylierten Chitosanen und hydroxyalkylierten Chitosanen als Zusatzstoffe zur Verbesserung der filmbildenden Eigenschaften von Polyvinylpyrrolidon-Vinylacetat-Copolymeren.

## Claims

1. Hair-cosmetic formulations containing
a) cationic biopolymers selected from the group consisting of chitosans, quaternized chitosans, alkylated chitosans and hydroxyalkylated chitosans and
b) polyvinyl pyrrolidone/vinyl acetate copolymers
in a ratio by weight of 1:10 to 1:45.

2. Use of cationic biopolymers selected from the group consisting of chitosans, quaternized chitosans, alkylated chitosans and hydroxyalkylated chitosans as additives for improving the film-forming properties of polyvinyl pyrrolidone/vinyl acetate copolymers.

## Revendications

1. Préparations cosmétiques pour les cheveux contenant :
a. des biopolymères cationiques sélectionnés dans le groupe composé par les chitosanes, les chitosanes quaternisés, les chitosanes alcoylés et les chitosanes hydroxy-alcoylés et
b. des copolymères polyvinylpyrrolidone/acétate de vinyle
dans un rapport pondérals allant de 1 :10 à 1 :45.

2. Utilisation de biopolymères cationiques sélectionnés dans le groupe composé par les chitosanes, les chitosanes quaternisés, les chitosanes alcoylés et les chitosans hydroxy-alcoylés comme additifs en vue de l'amélioration des propriétés filmogènes de copolymères polyvinylpyrrolidone/acétate de vinyle.
